# EUROPEAN PATENT SPECIFICATION

(11) **EP 4 385 405 B1**
(45) Date of publication and mention of the grant of the patent: **11.02.2026**
(21) Application number: 23217062.1
(22) Date of filing: 15.12.2023
(51) Int. Cl.: A61B 5/0538, A61B 5/06, A61B 5/367, A61B 5/00, A61B 34/20, A61B 17/00, A61B 34/10

(54) **CATHETER WITH COAXIAL COILS FOR POSITION SENSING AND TISSUE IMPEDANCE MEASUREMENT**
KATHETER MIT KOAXIALEN SPULEN ZUR POSITIONSBESTIMMUNG UND GEWEBEIMPEDANZMESSUNG
CATHÉTER AVEC BOBINES COAXIALES POUR DÉTECTION DE POSITION ET MESURE D'IMPÉDANCE TISSULAIRE

(30) Priority: 16.12.2022 US 202218083422
(43) Date of publication of application: 19.06.2024
(73) Proprietor: Biosense Webster (Israel) Ltd., 2066717 Yokneam (IL)
(72) Inventor: BOTZER, Lior, 2066717 Yokneam (IL); BAR-TAL, Meir, 2066717 Yokneam (IL); KDOSHAI-SCLIAR, Hen, 2066717 Yokneam (IL)
(74) Representative: Carpmaels & Ransford LLP

(56) References cited:
- EP-B1- 3 254 614
- US-A1- 2010 079 158

## Description

### TECHNICAL FIELD

The present disclosure relates generally to the field of medical devices, and particularly to sensors and sensing devices usable with catheters to detect various signals associated with medical procedures.

### BACKGROUND

Catheters may include tissue sensing electrodes (e.g., ECG electrodes) for sensing a parameter of interest and magnetic position sensors to accurately track location of the catheter. The magnetic position sensor is a coil through which a magnetic field may be generated. The coil is dedicated to sensing the position of the catheter in the body. Presently, such dedicated coils are only used for magnetic position sensing.

Catheters that do not include magnetic position sensors use Active Current Location (ACL) components and systems, to track the location of the catheter. ACL relies upon injection of a small electrical current from an electrode on the catheter inside the body so that patch electrodes outside the body can be used to triangulate the location of the catheter electrode from measurements of the resulting impedance of the electrical current through the body. Example ACL systems include those detailed in US Patent No. 7,756,576. 7,869,865, 7,848,787, and 8,456,182, and are typically associated with CARTO^{®} systems and procedures, from Biosense Webster, the owner of this application. However, when compared to magnetic position sensors, ACL systems drive current and calculate positions based on impedance, and accordingly, are much less accurate than magnetic position sensors.

EP3254614A1 describes an apparatus that includes an expandable structure, configured for insertion into a body of a subject, and a plurality of conducting elements coupled to the expandable structure. Each of the conducting elements comprises a respective coil and has an insulated portion that is electrically insulated from tissue of the subject, and an uninsulated portion configured to exchange signals with the tissue, while in contact with the tissue.

US2010079158A1 describes a method that includes positioning body-electrodes in galvanic contact with a body of a patient and positioning a mapping-tool, having a mapping-electrode, in a plurality of regions in the body. The method further includes tracking the mapping-tool at different positions in each of the regions using a location-measuring system, and for each region, generating a respective set of calibration-currents between the body-electrodes and the mapping-electrode at the different positions in the region. A respective relation is derived for each region between the respective set of the calibration-currents and the different positions, and is used in determining the location of an investigation-tool in response to the different respective relations and investigation-tool-currents.

### SUMMARY OF THE DISCLOSURE

The present disclosed subject matter provides a sensor unit, typically used on a catheter, as outer coil extending along the catheter. The coil is formed of wire windings, with at least two of the windings uninsulated or exposed. These unexposed windings allow the sensor unit to perform the dual functions of sensing tissue impedance, also referred to as environmental impedance, based on detecting changes in flux from a generated magnetic field and sensing tissue signals, such as electrocardiogram (ECG) signals, sent as electrical impulses (signals) from the heart (e.g., heart wall) during contraction.

The present disclosed subject matter provides a sensor for a catheter comprising: a first coil at a location within the catheter and electrically insulated by the catheter, for outputting signals for determining the position of the first coil within the body; and, a second coil along the outside of the catheter, the second coil including an uninsulated portion of a predetermined number of windings configured to output 1) electrocardiogram (ECG) signals, and, 2) signals indicative of voltages corresponding to tissue impedance from a magnetic field through body tissues and/or fluids.

### BRIEF DESCRIPTION OF THE DRAWINGS

The present disclosure will be more fully understood from the following detailed description of embodiments thereof, taken together with the drawings, in which:
Fig. 1 is a schematic illustration of an electroanatomical mapping system for cardiac mapping, which includes a catheter on which the disclosed sensor units are deployed;
Fig. 2 is an illustration of a sensor unit disposed on a portion of a catheter, in accordance with an example of the present disclosure; and
Fig. 3 is a flow diagram of example processes for acquiring data from the disclosed sensor units to produce various outputs.

### DETAILED DESCRIPTION OF EXAMPLES OVERVIEW

The present disclosed subject matter provides sensor units or sensors, for example, operational with catheters, such as a focal catheter or other catheter, which is used, for example, in procedures such as electroanatomical mapping of a body organ, such as the heart. The electroanatomical mapping may be part of a CARTO^{®} procedure. The sensor units are positioned on the catheter, at various known distances from each other, and typically the same distance from each other. Each of the sensor units includes an inner coil, inside of the catheter, which is electrically insulated, for example, by the catheter, from contacting tissue, blood or other bodily fluids, which, when in the body, and at least a partially uninsulated outer coil, outside of the catheter, which when in the body is directly exposed to and potentially in contact with tissue, blood and other bodily fluids.

The insulated inner coil functions, for example, as a magnetic position sensor. The inner coil is such that in the presence of a magnetic field, the inner coil outputs signals, which are usable to ascertain the location and/or orientation of the catheter on which the sensor units are disposed.

The uninsulated outer coil performs a plurality of functions. A first function includes a tissue sensing electrode, where the outer coil exchanges signals with tissue, such as by acquiring electrocardiogram (ECG) signals from tissue, such as cardiac tissue. The ECG signals are produced by electrical currents in the heart which cause contractions in the heart walls, and create different potentials through the body. The outer coil outputs these ECG signals to a processor or computer, which, for example, uses these signals in creating a cardiac map, for example, as part of a CARTO^{®} procedure, or performs other analysis thereof.

A second function includes operation as a conductivity sensor, which is affected by impedance, for example, electrical impedance or environmental impedance, as a generated electric field passes through tissue/blood, resulting in changes in the impedance, based on the various tissue and/or fluid that the electric field passes through. The detected (sensed) conductivity values of the electric flux are output by the outer coil as complex voltages and current to a processor or computer. The output voltages correspond to impedance in tissues and/or fluids and can further be used to provide indication on proximity to the tissue, and are indicative of various aspects of tissues, such as of tissue types, fluid types, e.g., blood or other bodily fluids, in the body location where the catheter is operating, as analyzed by the processor and/or computer. Additionally, as the outer uninsulated coil operates with the aforementioned generated magnetic field, it may also function as a magnetic position sensor, similar to the magnetic position sensor operation of the inner coil.

Since the inner and outer coils operate with a generated magnetic field, for example, from magnetic field generators proximate (e.g., underneath or in the vicinity of the subject in which the catheter is operating) to the catheter with the sensor units, the inner and outer coils typically do not drive current, for to do so would interfere with the generated magnetic field that the inner and outer coils are used to detect. The plurality of functions performed by the outer uninsulated coil may be performed contemporaneously, and in some cases, simultaneously.

Additionally, as the outer coil performs multiple functions, it replaces individual sensors previously used for each of the functions, therefore, reducing complexity of the catheter and system associated therewith.

The present disclosure also processes signals received from the above-described inner coil and outer coil, a plurality of outputs, which are used by a processor and/or computer to construct an electroanatomical map. These outputs include a plurality of first outputs from the outer coil, which indicate the electrical activity of the tissue (ECG), and a plurality of second outputs, also from the outer coil, which indicate the respective induced voltage differences across the outer coil corresponding to current that is leaking between individual windings in the other exposed coil. This leaked current is related to tissue/material surrounding the outer coils.

There are also a plurality of third outputs, at least from the inner coil, which indicate the proximity to certain tissue or the position of each of the sensor units, and accordingly, the catheter. In some instances, the outer coil may also function to produce the aforementioned third outputs for position of the catheter and/or the sensor units thereon.

### APPARATUS DESCRIPTION

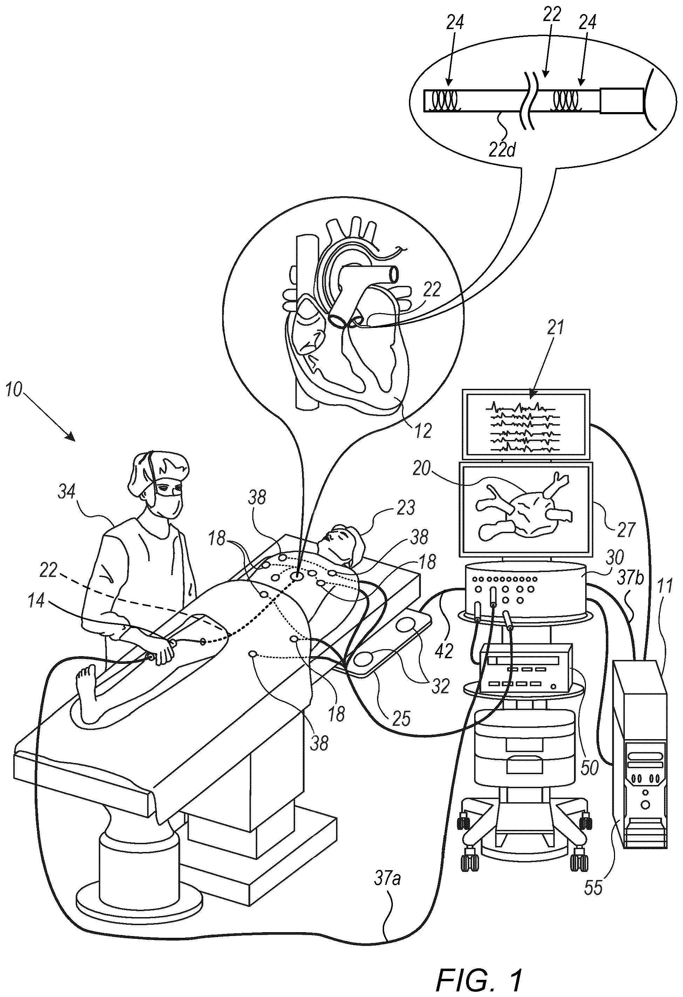

Reference is made to FIG. 1 showing an example catheter-based electrophysiology mapping and ablation system 10. System 10 includes multiple catheters, which are percutaneously inserted by physician 34 through the patient's vascular system into a chamber or vascular structure of a heart 12. Typically, a delivery sheath 14, one or more catheters, represented by the catheter 22 is inserted into the left or right atrium near a desired location in heart 12. Thereafter, a plurality of catheters can be inserted into the delivery sheath catheter so as to arrive at the desired location. The plurality of catheters may include catheters dedicated for sensing Intracardiac Electrogram (IEGM) signals, catheters dedicated for ablating and/or catheters dedicated for both sensing and ablating.

An example catheter 22 such as a focal catheter, is used to perform an electroanatomical mapping of the heart 12 of a subject 23. During the mapping procedure, the distal end 22d of the catheter 22, which comprises one or more sensor units or sensors 24, is inserted into heart 12.

For IEGM procedures, the physician 34 brings a distal tip of the catheter 22 into contact with the heart wall for sensing a target site in the heart 12. For ablation, the physician 34 similarly brings a distal end of an ablation catheter to a target site for ablating.

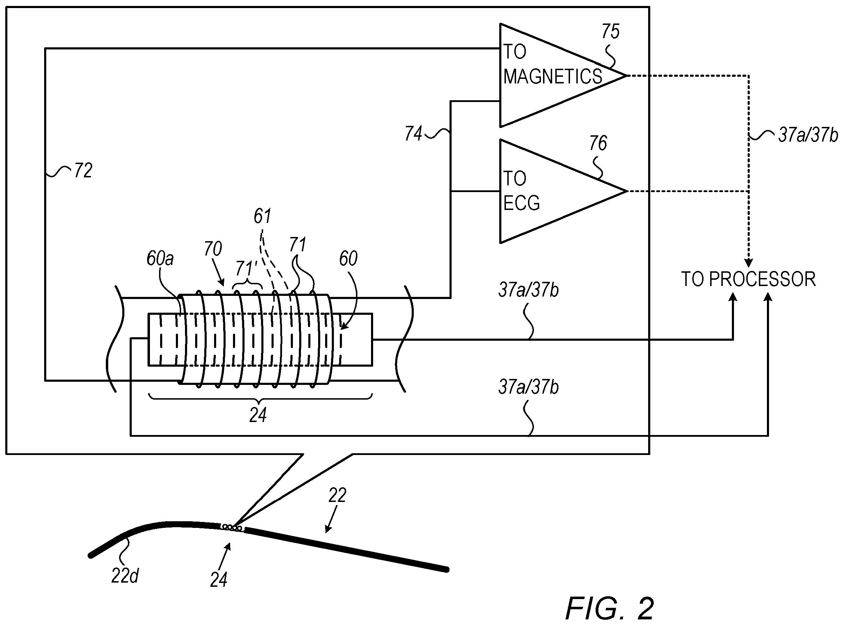

The focal catheter 22 includes, for example, sensor units 24, which are shown in detail in Fig. 2, and are described in detail below. Each sensor unit 24, for example, functions as a tissue sensing electrode, to detect electrocardiogram (ECG) signals, as well as functioning as inductance sensors, to detect changes in magnetic flux, from the generated magnetic fields (generated by magnetic-field generators 32, i.e., coils or magnetic radiators, located underneath subject 23 or otherwise in the vicinity of the subject 23), and output these detected changes as voltages, e.g., voltage changes. The detected magnetic field (magnetic flux) changes, as voltages, are input into the processor (not shown) in the workstation 55, which is programmed, for example, to analyze tissue impedance (Tissue Proximity Indicator (TPI)), for example, based the received voltage changes. These received voltages are used to deduce information about tissues and/or fluids proximate to the catheter 22 and/or each sensing unit 24, as well as to detect the position of the catheter 22 and/or sensor unit 24 on the catheter, in the body, for example, the heart 12.

The sensor units 24 communicate with the processor of the workstation 55. The processor, for example, is programmed to analyze the various signals captured by the sensor unit 24. The received signals and magnetic field data are transmitted from the sensor unit 24 to the processor, for example, over one or more lines 37a, 37b.

Typically, the processor comprises a general-purpose computer, which is programmed in software to carry out the functions described herein. The software may be downloaded to the computer in electronic form, over a network, for example, or it may, alternatively or additionally, be provided and/or stored on non-transitory tangible media, such as magnetic, optical, or electronic memory.

A signal generator, for example, in the patient interface unit (PIU) 30, typically causes the generators 32 to generate the magnetic field by supplying an alternating current (e.g., over the line 42) to the generators 32. The generated magnetic field induces voltage differences across the inner 60 and outer 70 coils (Fig. 2) (the inner 60 and outer 70 coils also known as conducting elements, inner and outer, respectively) detailed below, of the sensor unit 24. For example, the induced voltage differences (output by the inner 60 and outer 70 coils, respectively) are received by the PIU 30 and then the processor in the workstation 55 (via the lines 37a, 37b), and, based on the induced voltages, the processor ascertains the position of at least the inner coil 60, and in some cases, the also outer coil 70, as well as tissue impedance (also known as environmental impedance) measurements (tissue proximity indicators (TPI)) from the outer coil 70 (for the location in the body of the catheter 22).

For example, the processor may be programmed to compare the voltages, representative of various tissue and/or fluid impedances, to voltages in a database(s), look up table (LUT), other stored values, or references, or the like, to determine the tissue and/or fluid type. The tissue and/or fluid types may be displayed on the monitor 21.

The processor is typically also programmed to construct an electroanatomical map 20 of the heart 12 (the body location where the catheter 22 operates), based on the ECG signals (which indicate the electrical activity of the intracardiac tissue) and the voltages received from the helical conducting elements (which indicate the respective locations of the sources of the ECG signals). Such a map 20 may be displayed on a monitor 27 for viewing by physician 34, and/or stored for later analysis.

The sensor units 24 may be arranged at intervals of various distances apart from each other, for example, approximately 1 mm to 4 mm. For example, the distances between the sensor units 24 are the same, and the locations of the sensor units 24 on the catheter 22 is known.

A recorder 11 displays electrograms 21 captured with body surface ECG electrodes 18 and intracardiac electrograms (IEGM) captured with electrodes 24 of catheter 22. The recorder 11 may include pacing capability for pacing the heart rhythm and/or may be electrically connected to a standalone pacer.

System 10 may include an ablation energy generator 50 that is adapted to conduct ablative energy to one or more of electrodes at a distal tip of a catheter configured for ablating. Energy produced by ablation energy generator 50 may include, but is not limited to, radiofrequency (RF) energy or pulsed-field ablation (PFA) energy, including monopolar or bipolar high-voltage DC pulses as may be used to effect irreversible electroporation (IRE), or combinations thereof.

The patient interface unit (PIU) 30 is an interface configured to establish electrical communication between catheters, electrophysiological equipment, power supply and a workstation 55 for controlling operation of system 10. Electrophysiological equipment of system 10 may include for example, multiple catheters, location pad 25, body surface ECG electrodes 18, electrode patches 38, ablation energy generator 50, and recorder 11. Optionally, PIU 30 additionally includes processing capability for implementing real-time computations of location of the catheters and for performing ECG calculations.

Workstation 55 includes memory, processor unit with memory or storage with appropriate operating software loaded therein, and user interface capability. Workstation 55 may provide multiple functions, optionally including (1) modeling the endocardial anatomy in three-dimensions (3D) and rendering the model or anatomical map 20 for display on a display device 27, (2) displaying on display device 27 activation sequences (or other data) compiled from recorded electrograms 21 in representative visual indicia or imagery superimposed on the rendered anatomical map 20, (3) displaying real-time location and orientation of multiple catheters within the heart chamber, and (4) displaying on display device 27 sites of interest such as places where ablation energy has been applied. One commercial product embodying elements of the system 10 is available as the CARTO^{™} 3 System, available from Biosense Webster, Inc., 31A Technology Drive, Irvine, CA 92618.

Turning also to Fig. 2, where a section of the catheter 22 is shown with a sensor unit 24. The sensor unit 24 is formed of an inner conducting element, for example, a coil 60 (also known as the inner coil) of wire(s) 61, inside of the catheter 22, and, an outer conducting element, for example, a coil 70 (also known as the outer coil), outside of the catheter 22, also formed, for example, of a coiled electrically conductive wire or wires 71 (or windings or helical windings), with at least a portion of the wires 71' of the outer coil 70 being uninsulated and exposed. For example, the uninsulated or exposed portion of the outer coil 70 includes at least two windings of the outer coil 70. Accordingly, the inner coil 60, which is, for example, fully insulated, is affected typically by the flux of the magnetic field, as generated by the generators 32. The outer coil 70 is affected also by magnetic flux provided by the magnetic radiators 25 and changes in conductivity due to its direct physical contact with the external environment (e.g., blood or tissue). The difference in the change of the flux of the magnetic field is an indication of conductivity changes. For example, the difference in the change of flux of the magnetic field together with the known location of the coil, and the magnetic field, is convertible to conductivity.

The inner coil 60 (of wire(s) 61) is electrically insulated, for example, by the catheter 22 itself, or other insulation material 60a inside of the catheter 22. The inner coil 60 includes, for example, a dedicated voltage channel, which includes the line 37a, 37b, so that the induced voltage differences caused by the generated magnetic field (e.g., the magnetic field generates a potential on the inner coil 60), are relayed to the processor (not shown) in the workstation 55. The processor analyzes the received voltages (potentials) and ascertains, or otherwise determines, the position of the inner coil 60 within the body, for example, within the heart 12.

The outer coil 70, including, for example, the at least two uninsulated windings (wire portion 71'), is positioned along the outer surface of the catheter 22. The outer coil 70 and inner coil 60 are, according to the invention, arranged so as to be coaxial and in embodiments also concentric with respect to each other. According to the invention, the outer coil 70 extends over (and 30 along) at least a portion of the inner coil 60 and in embodiments is shifted with known bias, so as to extend within the span of the inner coil 60 (e.g., the inner coil 60 typically extends longitudinally beyond the outer coil 70). The outer coil 70 may be wound at a first helix angle for the outer coil winding (wire(s) 71) while the inner coil 60 may be wound (wire(s) 61) at a second helix angle for the inner coil winding being different from the first helix winding angle. The two known but different first and second helical winding angles will allow for the inner coil 60 and outer coil 70 to act as a dual-axis magnetic location sensor.

The outer coil 70 includes a dedicated voltage channel (for measuring potential), which includes sublines 72, 74 for signals representative of received magnetics by operational amplifier (op-amp) 75, and signals representative of received ECG signals, by op-amp 76, which communicate with the processor in the workstation 55 via the lines 37a, 37b. The outer coil 70, by virtue of the wire(s) 71 which form it, include a portion 71' being uninsulated and otherwise exposed, contacts body tissues and/or fluids, such as blood, and the like.

The outer coil 70, is formed, for example, from a single coil, or multiple coils joined to form the outer coil 70, with an insulated wire(s). The wire(s) 71 are wound in a series of helical windings or turns around the catheter 22. A portion of the insultation is removed from the wire(s), for example, mechanically by a laser, to expose portions of the wire(s). The exposure of the wire portions is made in a manner that does not lead to contact between the windings. Accordingly, a partially exposed outer coil 70 may function as a position sensor, similar to that of the inner coil 60.

The outer coil 70, for example, via its at least two, and typically, two or more uninsulated or exposed windings, performs a plurality of functions. One function of the outer coil 70 is as an electrode, where the outer coil 70 exchanges signals with tissue, such as by acquiring electrocardiogram (ECG) signals from tissue or to drive electrical signals from the outer coil 70 into the tissues such as for pacing. Another function is a magnetic field sensor unit (sensor) 24 or flux sensor, which detects the magnetic field generated (by magnetic field generators 32) as received by the outer coil 70, and output by the outer coil 70 as voltage changes resulting from impedance from tissues and/or fluids with respect to the generated magnetic field 30. The tissue impedance values, and accordingly, the output voltages, are different for each type of tissue and/or fluid, and are, for example, used by the processor 32 to determine one or more of tissue types, tissue boundaries, locations of certain tissue, and/or fluids, and the like.

The outer coil 70 is such that different frequency bands may be used for analyzing position and tissue impedances (e.g., parameters) through the outer coil 70 and the lines extending from the wires 71 of the outer coil 70. For example, ECG may occupy DC to 1 KHz while magnetic related voltage, and in some cases, also magnetic position sensing, may use, for example, approximately 2 KHz to 8 KHz. Since the frequency spectra for ECG (electrical activity sensing) and position sensing is different, the two signals do not overlap, and therefore, may be concurrently transmitted by the same wire.

The outer coil 70 may also function as a magnetic position sensor, identical or similar to that of inner coil 60, as detailed above. Based on the detected outer coil 70, the position of the outer coil 70 and/or the sensor unit 24 in the body, for example, the heart 12, is determined. When the outer coil 70 is used as a position sensor (as detailed below), the inner coil 60 serves as a reference.

Alternately, the inner coil 60 need not be present, provided that there is a known physical relationship between the external leaky coil and an external sensor, such as the sensor unit 24.

The outer coil 70 is such that at least two, and typically, two or more windings (wire portion 71'), which are uninsulated and otherwise exposed, are needed. This is because with only one exposed winding, the outer coil 70 would only be usable for position sensing and ECG, due to a single exposed winding having a high impedance. As a result, outer coils with only a single winding, which is uninsulated and exposed, would require an active current location (ACL) location sensing system.

The disclosed outer coil 70 is designed with two or more uninsulated and exposed windings, so that the outer coil 70 can operate absent ACL location sensing systems, and, as such current does not need to be driven through the outer coil 70 (or the inner coil 60), which would have the potential to interfere with the magnetic fields or the ECG signals from the generators 30 and comes with a need for specific hardware that generates and measures the current. Moreover, by having at least two uninsulated and exposed windings of the outer coil 70, position calculations are not based on impedance, as is the case with ACL systems.

The windings of the outer coil 70, and at least the uninsulated or exposed windings (wire portion 71) are, for example, spaced from each other at least at a minimum distance, to avoid interference, including electrical interference, which is a potential cause of short circuiting. This minimum distance is, for example, at least approximately 0.5 mm. The distance between the windings affects sensitivity. For example, a larger distance or spacing between the individual windings renders the outer coil 70 less sensitive, when compared to the outer coil 70 having a shorter separation distance between windings. The number of windings (turns) of the coil may also affect sensitivity. Also, the material of the coil 70 affects the sensitivity, as well as the distance between the windings.

The leakage current between the windings of the outer coil 70 is affected by the gradient voltage on the outer coil 70. Therefore, different materials will generate different voltages and current leakage, the higher the voltage the larger the sensitivity to tissue related impedance. Materials for the wire coils 70 include, for example, medical grade Stainless Steel, and platinum wire. The wire can be also coated at intermediate locations between one or more windings, to achieve better/tailored sensitivity in specific areas of the exposed wire.

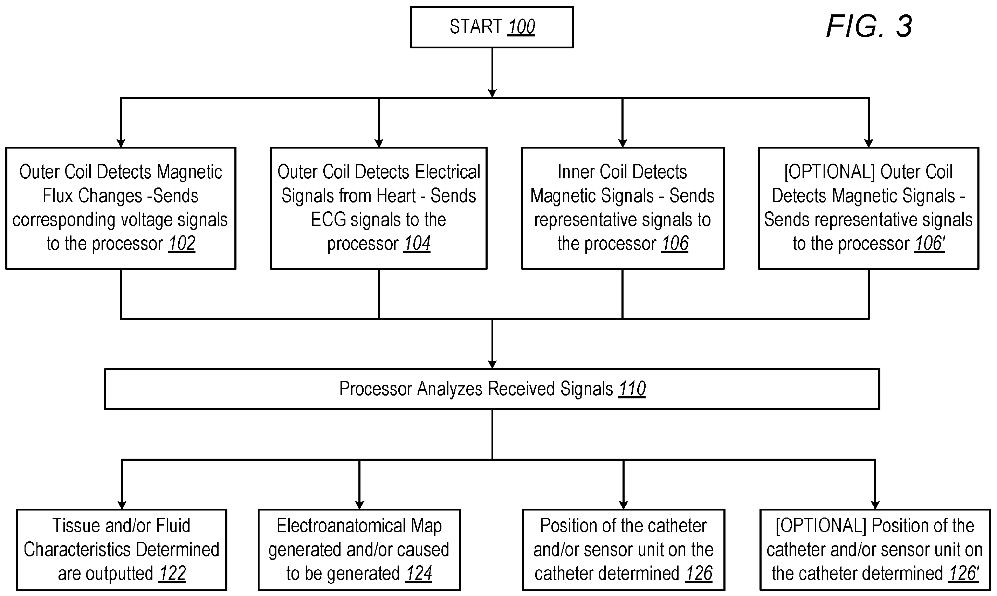

Fig. 3 is a flow diagram of example processes performed by the processor, based on signals received from the outer coil 70, and in some cases, also the inner coil 60. The processes are, for example, performed automatically and in real time, and may include manual subprocesses. The process may be performed as long as desired.

The process begins at a START block 100, where, for example, the catheter 22 is deployed in the subject 23, for example, in the heart 12. Additionally, a magnetic field is being generated by the generators 32, as controlled via the workstation 55.

At block 102, signals corresponding to the changes in magnetic flux through the various tissues where the catheter 22 is operating, represented voltage signals, indicative of the voltage changes from the changing magnetic flux, as detected by the uninsulated and exposed windings (wire portion 71') of the outer coil 70, are sent (outputted) to the processor 32. At block 110, the processor 32 analyzes these signals, for example, based the received voltage changes from the outer coil 70. These received voltages changes are used to deduce information about tissues and/or fluids proximate to the catheter 22 and/or each sensing unit 24, as well as to detect the position of the catheter 22 and/or sensor unit 24 on the catheter, in the body, for example, the heart 12. At block 122, the processor outputs tissue and/or fluid characteristics, such as tissue impedance value or tissue or fluid types, tissue and/or fluid boundaries, and the like.

At block 104, ECG signals are received by the outer coil 70 and sent (outputted) to the processor. The processor analyzes these signals, at block 110, and, at block 124, generates, or otherwise produces, or causes the production of an electro anatomical map of the heart 12.

At block 106 the inner coil 60 detects the generated magnetic field, and sends corresponding signal to the processor. The processor analyzes these received signals at block 110, and at block 126, determines a position of the catheter 22 and/or the sensor unit 24 thereon.

In an optional process at block 106' the outer coil 70 detects the generated magnetic field, and sends corresponding signal to the processor. The processor analyzes these received signals at block 110, and at block 126', determines a position of the catheter 22 and/or the sensor unit 24 thereon.

Although the examples described herein mainly address catheters for cardiac procedures, the disclosed sensor units may also be used with instrumentation associated with neurological procedures, such as in TruDi^{™} procedures for brain tumor border identification, and other tissue and/or fluid identifications in the body. The coils described herein may also be produced with flex PCB techniques.

It will be appreciated by persons skilled in the art that the present disclosure is not limited to what has been particularly shown and described hereinabove. The scope of the invention is set out by the appended claims.

## Claims

1. A catheter (22) comprising a sensor (24), the sensor (24) comprising:
a first coil (60) at a location within the catheter (22) and electrically insulated by the catheter (22), the first coil (60) for outputting signals for determining the position of the first coil (60) within the body; and
a second coil (70) coaxial with respect to, and extending over at least a portion of, the first coil (60), the second coil (70) along the outside of the catheter (22), the second coil (70) including an uninsulated portion of a predetermined number of windings configured to output 1) electrocardiogram (ECG) signals, and, 2) signals indicative of voltages corresponding to tissue impedance from a magnetic field through body tissues and/or fluids.

2. The catheter (22) of claim 1, wherein the second coil (70) is additionally configured to output signals for determining the position of the second coil (70) within the body.

3. The catheter (22) of claim 1 or claim 2, wherein the predetermined number of windings includes at least two.

4. The catheter (22) of any preceding claim, wherein the first coil (60) and the second coil (70) are concentric with each other.

5. The catheter (22) of any preceding claim, wherein the second coil (70) extends within the span of the first coil (60).

6. The catheter (22) of any preceding claim, additionally comprising: a processor in communication with the second coil (70) for receiving signals indicative of voltages corresponding to impedance from the magnetic field through body tissues and/or fluids, and analyzing the received signals to determine the body tissues and/or fluids proximate to the sensor (24).

7. The catheter (22) of any preceding claim, additionally comprising: a processor in communication with the second coil (70) for receiving EGG signals for constructing an electroanatomical map of the body traveled by the catheter (22).

8. The catheter (22) of any preceding claim, wherein the second coil (70) includes one or more wires wound helically around the catheter (22) such that there are at least two windings.

9. The catheter (22) of claim 4, wherein the at least two windings are at a predetermined distance from each other for avoiding short circuiting between the windings.

10. The catheter (22) of claim 9, wherein the predetermined distance is at least 0.5 mm.

11. A method for obtaining data in a body comprising:
providing a catheter (22) comprising at least one sensor (24), the at least one sensor (24) comprising:
a first coil (60) at a location within the catheter (22) and electrically insulated by the catheter (22), the first coil (60) for outputting signals for determining the position of the first coil (60) within the body; and
a second coil (70) coaxial with respect to, and extending over at least a portion of, the first coil (60), the second coil (70) along the a outside of the catheter (22), the second coil (70) including an uninsulated portion of a predetermined number of windings configured to output 1) electrocardiogram (ECG) signals, and, 2) signals indicative of voltages corresponding to impedance from a generated magnetic field through body tissues and/or fluids;
the first coil (60) and the second coil (70) outputting signals, for receipt as input by a processor (32); and
the processor (32) programmed to analyze the input signals and perform one or more of: 1) generating an electroanatomical map of the body location where the catheter (22) operates, and/or 2) determining characteristics of tissue and/or fluid of the body location where the catheter (22) operates.

## Patentansprüche

1. Katheter (22), der einen Sensor (24) umfasst, wobei der Sensor (24) umfasst:
eine erste Spule (60) an einer Stelle innerhalb des Katheters (22) und durch den Katheter (22) elektrisch isoliert, wobei die erste Spule (60) zum Ausgeben von Signalen zum Bestimmen der Position der ersten Spule (60) innerhalb des Körpers dient; und
eine zweite Spule (70), die koaxial zu der ersten Spule (60) angeordnet ist und sich über mindestens einen Abschnitt davon erstreckt, wobei sich die zweite Spule (70) entlang der Außenseite des Katheters (22) befindet, wobei die zweite Spule (70) einen nicht isolierten Abschnitt mit einer vorbestimmten Anzahl von Windungen einschließt, der konfiguriert ist, um 1) Elektrokardiogramm-(EKG)-Signale und 2) Signale, die Spannungen entsprechend der Gewebeimpedanz von einem Magnetfeld durch Körpergewebe und/oder Körperflüssigkeiten anzeigen, auszugeben.

2. Katheter (22) nach Anspruch 1, wobei die zweite Spule (70) zusätzlich konfiguriert ist, um Signale zum Bestimmen der Position der zweiten Spule (70) innerhalb des Körpers auszugeben.

3. Katheter (22) nach Anspruch 1 oder 2, wobei die vorbestimmte Anzahl von Windungen mindestens zwei einschließt.

4. Katheter (22) nach einem der vorstehenden Ansprüche, wobei die erste Spule (60) und die zweite Spule (70) konzentrisch zueinander sind.

5. Katheter (22) nach einem der vorstehenden Ansprüche, wobei sich die zweite Spule (70) innerhalb der Spannweite der ersten Spule (60) erstreckt.

6. Katheter (22) nach einem der vorstehenden Ansprüche, zusätzlich umfassend: einen Prozessor in Kommunikation mit der zweiten Spule (70) zum Empfangen von Signalen, die Spannungen entsprechend der Impedanz aus dem Magnetfeld durch Körpergewebe und/oder Körperflüssigkeiten anzeigen, und zum Analysieren der empfangenen Signale, um die Körpergewebe und/oder Körperflüssigkeiten in der Nähe des Sensors (24) zu bestimmen.

7. Katheter (22) nach einem der vorstehenden Ansprüche, zusätzlich umfassend: einen Prozessor in Kommunikation mit der zweiten Spule (70) zum Empfangen von EGG-Signalen zum Konstruieren einer elektroanatomischen Karte des vom Katheter (22) durchlaufenen Körpers.

8. Katheter (22) nach einem der vorstehenden Ansprüche, wobei die zweite Spule (70) einen oder mehrere Drähte einschließt, die spiralförmig um den Katheter (22) gewickelt sind, so dass mindestens zwei Windungen vorhanden sind.

9. Katheter (22) nach Anspruch 4, wobei die mindestens zwei Windungen in einem vorbestimmten Abstand voneinander angeordnet sind, um einen Kurzschluss zwischen den Windungen zu vermeiden.

10. Katheter (22) nach Anspruch 9, wobei der vorbestimmte Abstand mindestens 0,5 mm beträgt.

11. Verfahren zum Erhalten von Daten in einem Körper, umfassend:
Bereitstellen eines Katheters (22), der mindestens einen Sensor (24) umfasst, wobei der mindestens eine Sensor (24) umfasst:
eine erste Spule (60) an einer Stelle innerhalb des Katheters (22) und durch den Katheter (22) elektrisch isoliert, wobei die erste Spule (60) zum Ausgeben von Signalen zum Bestimmen der Position der ersten Spule (60) innerhalb des Körpers dient; und
eine zweite Spule (70), die koaxial zu der ersten Spule (60) angeordnet ist und sich über mindestens einen Abschnitt davon erstreckt, wobei sich die zweite Spule (70) entlang einer Außenseite des Katheters (22) befindet, wobei die zweite Spule (70) einen nicht isolierten Abschnitt mit einer vorbestimmten Anzahl von Windungen einschließt, der konfiguriert ist, um 1) Elektrokardiogramm-(EKG)-Signale und 2) Signale, die Spannungen anzeigen, welche einer Impedanz von einem erzeugten Magnetfeld durch Körpergewebe und/oder Körperflüssigkeiten entsprechen, auszugeben; die erste Spule (60) und die zweite Spule (70), die Signale ausgeben, zum Empfang als Eingabe durch einen Prozessor (32); und
den Prozessor (32), der programmiert ist, um die Eingangssignale zu analysieren und eines oder mehrere der folgenden durchzuführen: 1) Erzeugen einer elektroanatomischen Karte der Körperstelle, an der der Katheter (22) arbeitet, und/oder 2) Bestimmen von Eigenschaften von Gewebe und/oder Flüssigkeit der Körperstelle, an der der Katheter (22) arbeitet.

## Revendications

1. Cathéter (22) comprenant un capteur (24), le capteur (24) comprenant :
une première bobine (60) au niveau d'une localisation au sein du cathéter (22) et électriquement isolée par le cathéter (22), la première bobine (60) permettant de délivrer en sortie des signaux permettant de déterminer la position de la première bobine (60) au sein du corps ; et
une seconde bobine (70) coaxiale par rapport à, et s'étendant pardessus au moins une partie de, la première bobine (60), la seconde bobine (70) étant le long de l'extérieur du cathéter (22), la seconde bobine (70) comportant une partie non isolée d'un nombre prédéterminé d'enroulements configurée pour délivrer en sortie 1) des signaux d'électrocardiogramme (ECG), et, 2) des signaux indiquant des tensions correspondant à une impédance de tissu provenant d'un champ magnétique à travers des tissus et/ou fluides corporels.

2. Cathéter (22) selon la revendication 1, dans lequel la seconde bobine (70) est en outre configurée pour délivrer en sortie des signaux permettant de déterminer la position de la seconde bobine (70) au sein du corps.

3. Cathéter (22) selon la revendication 1 ou la revendication 2, dans lequel le nombre prédéterminé d'enroulements comporte au moins deux.

4. Cathéter (22) selon l'une quelconque revendication précédente, dans lequel la première bobine (60) et la seconde bobine (70) sont concentriques l'une par rapport à l'autre.

5. Cathéter (22) selon l'une quelconque revendication précédente, dans lequel la seconde bobine (70) s'étend au sein de la longueur de la première bobine (60).

6. Cathéter (22) selon l'une quelconque revendication précédente, comprenant en outre : un processeur en communication avec la seconde bobine (70) permettant de recevoir des signaux indiquant des tensions correspondant à une impédance provenant du champ magnétique à travers des tissus et/ou fluides corporels, et d'analyser les signaux reçus pour déterminer les tissus et/ou fluides corporels à proximité du capteur (24).

7. Cathéter (22) selon l'une quelconque revendication précédente, comprenant en outre : un processeur en communication avec la seconde bobine (70) permettant de recevoir des signaux EGG permettant de construire une carte électroanatomique du corps parcouru par le cathéter (22).

8. Cathéter (22) selon l'une quelconque revendication précédente, dans lequel la seconde bobine (70) comporte un ou plusieurs fils enroulés en spirale autour du cathéter (22) de telle sorte qu'il y a au moins deux enroulements.

9. Cathéter (22) selon la revendication 4, dans lequel les au moins deux enroulements sont à une distance prédéterminée l'un de l'autre pour éviter un court-circuitage entre les enroulements.

10. Cathéter (22) selon la revendication 9, dans lequel la distance prédéterminée est d'au moins 0,5 mm.

11. Procédé permettant d'obtenir des données dans un corps comprenant :
la fourniture d'un cathéter (22) comprenant au moins un capteur (24), l'au moins un capteur (24) comprenant :
une première bobine (60) au niveau d'une localisation au sein du cathéter (22) et électriquement isolée par le cathéter (22), la première bobine (60) permettant de délivrer en sortie des signaux permettant de déterminer la position de la première bobine (60) au sein du corps ; et
une seconde bobine (70) coaxiale par rapport à, et s'étendant pardessus au moins une partie de, la première bobine (60), la seconde bobine (70) étant le long d'un extérieur du cathéter (22), la seconde bobine (70) comportant une partie non isolée d'un nombre prédéterminé d'enroulements configurée pour délivrer en sortie 1) des signaux d'électrocardiogramme (ECG), et, 2) des signaux indiquant des tensions correspondant à une impédance provenant d'un champ magnétique généré à travers des tissus et/ou fluides corporels ; la première bobine (60) et la seconde bobine (70) délivrant en sortie des signaux, pour réception en guise d'entrée par un processeur (32) ; et
le processeur (32) programmé pour analyser les signaux d'entrée et mettre en œuvre une ou plusieurs parmi : 1) la génération d'une carte électroanatomique de la localisation de corps où le cathéter (22) fonctionne, et/ou 2) la détermination de caractéristiques de tissu et/ou de fluide de la localisation de corps où le cathéter (22) fonctionne.
